# FASCICULE DE BREVET EUROPEEN

(11) **EP 4 615 543 B1**
(45) Date de publication et mention de la délivrance du brevet: **14.10.2026**
(21) Numéro de dépôt: 23801432.8
(22) Date de dépôt: 06.11.2023
(51) Int. Cl.: A61M 11/06

(54) **DISPOSITIF ET PROCÉDÉ DE DÉTECTION D'UNE QUANTITÉ INSUFFISANTE DE LIQUIDE DANS UN NÉBULISEUR**
VORRICHTUNG UND VERFAHREN ZUR FESTSTELLUNG EINER UNGENÜGENDEN FLÜSSIGKEITSMENGE IN EINEM ZERSTÄUBER
DEVICE AND METHOD FOR DETECTING AN INSUFFICIENT QUANTITY OF LIQUID IN A NEBULISER

(30) Priorité: 07.11.2022 FR 2211580
(43) Date de publication de la demande: 17.09.2025
(73) Titulaire: ORIKIO, 29200 Brest (FR); SORBONNE UNIVERSITE, 75006 Paris (FR); Institut National de la Santé et de la Recherche Médicale, 75013 Paris (FR); Assistance Publique - Hôpitaux de Paris, 75012 Paris (FR)
(72) Inventeur: SIMILOWSKI, Thomas, 75006 PARIS (FR); MORELOT-PANZINI, Capucine, 75012 PARIS (FR); MENUT, Olivier, 29200 BREST (FR); GEFFROY, Vincent, 29200 BREST (FR)
(74) Mandataire: INNOV-GROUP
(86) Numéro de dépôt international: PCT/EP2023/080840
(87) Numéro de publication internationale: WO 2024/099966

(56) Documents cités:
- EP-A1- 1 026 482
- WO-A1-2014/149654
- WO-A1-2018/104805
- US-A- 4 776 990
- US-A1- 2012 017 894
- US-A1- 2018 161 531

## Description

### DOMAINE TECHNIQUE

Un nébuliseur permet de transformer un médicament liquide en aérosol, c'est-à-dire en très fines gouttelettes, destinées à atteindre les voies respiratoires. Parmi les nébuliseurs fréquemment utilisés, les nébuliseurs pneumatiques génèrent l'aérosol par l'action d'un gaz, envoyé sous pression, sur un liquide comportant un principe actif. Le gaz est usuellement de l'air. D'autres nébuliseurs utilisent des ultra-sons pour produire l'aérosol.

Quel que soit le mode de fonctionnement, le recours à un nébuliseur permet d'administrer un principe actif à un patient par voie respiratoire, sous la forme de petites gouttelettes formant un aérosol et qui sont inhalées par la bouche et/ou le nez. Le diamètre des gouttelettes est généralement inférieur à 10 µm.

Le liquide comportant le principe actif est contenu dans un réservoir. Au cours de l'utilisation du nébuliseur, la quantité de liquide, dans le réservoir, diminue. Certains utilisateurs peuvent ne pas avoir la faculté de détecter qu'un réservoir est vide ou la possibilité de stopper le fonctionnement du nébuliseur alors que le réservoir est vide, ou de demander une assistance lorsque le réservoir se vide. Il importe alors de ne pas laisser le nébuliseur sur le visage du patient, pour une raison de confort, et pour éviter, dans le cas d'un aérosol propulsé par du gaz, un gaspillage du gaz.

WO2018/104805 et US2012/017894 décrivent des dispositifs permettant d'exploiter une différence d'intensité du bruit lorsqu'un nébuliseur passe de l'état plein à l'état vide. Le document US2012017894 indique que le bruit est détecté dans une bande spectrale inférieure à 100 Hz.

On comprend qu'il est souhaitable de disposer d'un dispositif efficace, discret et simple d'utilisation, permettant de vérifier que le réservoir du nébuliseur soit suffisamment rempli. L'invention répond à ce besoin.

### EXPOSE DE L'INVENTION

Un premier objet de l'invention est un procédé de détection d'une quantité insuffisante de liquide dans un réservoir d'un nébuliseur, le nébuliseur étant configuré pour former un aérosol à partir du liquide présent dans le réservoir, de façon à administrer un principe actif, présent dans le liquide, à un utilisateur, le procédé comportant :
- a) détection, par un microphone, d'un son produit, en différents instants, par la formation de l'aérosol ;
- b) traitement du son détecté par une unité de traitement (3), à chaque instant de façon à en extraire au moins une caractéristique du son;
- c) en fonction de chaque caractéristique extraite lors de l'étape b), détection, par l'unité de traitement, d'une quantité insuffisante de liquide dans le réservoir pour l'administration du principe actif, le procédé étant caractérisé en ce que lors de l'étape b), la caractéristique du son est extraite dans une bande de fréquence comprise entre 3000 Hz et 20000 Hz.

L'étape c) peut comporter :
- c1) prise en compte d'au moins un critère préalablement mémorisé ;
- c2) confrontation de chaque caractéristique extraite lors de l'étape b) au critère pris en compte lors de la sous-étape c1).

Le traitement peut comporter :
- sélection d'au moins une bande de fréquence du son ;
- détermination d'une caractéristique dans la bande de fréquence sélectionnée.

Selon une possibilité,
- la caractéristique est une puissance spectrale ;
- le critère est un seuil de puissance spectrale, de façon que la quantité insuffisante de liquide est détectée lorsque la puissance spectrale, dans la bande de fréquence sélectionnée, franchit le seuil de puissance spectrale.

Selon une possibilité,
- la caractéristique est une variation de puissance spectrale ;
- le critère est un seuil de variation puissance spectrale, de façon que la quantité insuffisante de liquide est détectée lorsque la variation de puissance spectrale, dans la bande de fréquence sélectionnée, et dans un intervalle temporel prédéterminé, franchit le seuil de variation de la puissance spectrale.

Selon une possibilité,
- l'unité de traitement met en œuvre un algorithme d'intelligence artificielle à apprentissage supervisé ;
- l'algorithme d'intelligence artificielle à apprentissage supervisé est paramétré par une phase d'apprentissage prenant en compte des sons détectés en l'absence et en présence d'une anomalie ;

Un deuxième objet de l'invention est un dispositif de détection d'une quantité insuffisante de liquide dans un réservoir d'un nébuliseur, le nébuliseur étant configuré pour former un aérosol à partir du liquide présent dans le réservoir, le dispositif comportant :
- un microphone, configuré pour enregistrer des sons produits par la formation de l'aérosol;
- une unité de traitement, programmée pour recevoir les sons enregistrés par le microphone, et pour mettre en œuvre les étapes b) et c) d'un procédé selon le premier objet de l'invention.

L'invention sera mieux comprise à la lecture de l'exposé des exemples de réalisation présentés, dans la suite de la description, en lien avec les figures listées ci-dessous.

### FIGURES

La figure 1 représente une exemple de nébuliseur relié à un dispositif selon l'invention
La figure 2A représente une décomposition fréquentielle de sons produits au cours d'une utilisation d'un nébuliseur dont le réservoir présente une quantité suffisante de liquide.
La figure 2B représente une décomposition fréquentielle de sons produits au cours d'une utilisation d'un nébuliseur dont le réservoir présente une quantité insuffisante de liquide.
La figure 3 schématise des étapes de mises en œuvre d'un dispositif selon l'invention.

### EXPOSE DE MODES DE REALISATION PARTICULIERS

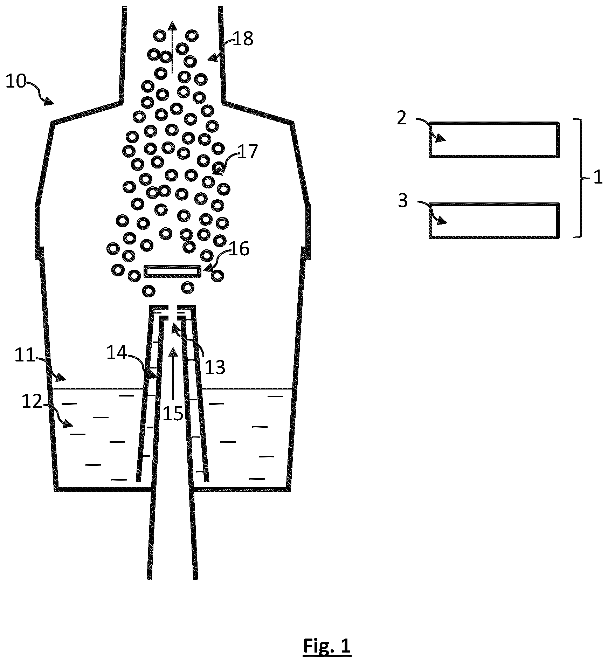

La figure 1 représente un nébuliseur 10 relié à un dispositif 1 selon l'invention. Le nébuliseur 10 comporte un réservoir 11, destiné à contenir un liquide 12. Le nébuliseur est configuré pour former un aérosol à partir du liquide 12.

Dans l'exemple représenté, le nébuliseur est un nébuliseur pneumatique, ce qui constitue un exemple particulier de mise en œuvre de l'invention. L'invention s'applique à d'autres types de nébuliseurs. Le nébuliseur comporte une admission d'air 13, à travers laquelle de l'air comprimé 15 est admis dans le réservoir. Le réservoir 11 comporte un circuit fluidique 14, permettant, au liquide présent dans le réservoir, d'affleurer l'admission d'air. L'air comprimé crée, à l'intérieur du réservoir, une dépression, qui permet un pompage du liquide 12 à travers le circuit fluidique 14, par effet venturi, vers l'admission d'air 13. Au niveau de l'admission d'air 13, l'air comprimé projette le liquide jusqu'à un déflecteur 16. Il en résulte une formation d'un aérosol 17, qui se propage vers une sortie 18.

La sortie 18 est reliée à une interface destiné à être appliquée contre le visage d'un utilisateur. L'interface peut être un masque respiratoire ou un embout buccal ou un embout narinaire.

Le dispositif comporte un microphone 2, relié à une unité de traitement 3. Le microphone est configuré pour enregistrer des sons produits par le nébuliseur au cours de son fonctionnement. Plus précisément, le microphone est configuré pour enregistrer le bruit généré par la nébulisation du liquide, à l'intérieur du réservoir 11.

L'unité de traitement 3 est programmée pour effectuer un traitement des sons collectés par le microphone 2. Plus précisément, il s'agit de sons produits par le liquide, présent dans le réservoir, au cours de la nébulisation.

Les inventeurs ont observé que lorsque la quantité de liquide, dans le réservoir, devient faible, une signature sonore caractéristique se produit. Cette signature sonore est attribuée à une fluctuation de la quantité de fluide pompé jusqu'à l'admission d'air 13. Lorsque la quantité de liquide est insuffisante, son administration au patient s'interrompt. Il est important de pouvoir le détecter. Ainsi, une analyse du son enregistré par le microphone 2 permet de détecter un éventuel défaut de remplissage du réservoir.

L'unité de traitement 3 est configurée pour :
- traiter les sons enregistrés par le microphone 2, de façon à en extraire des caractéristiques ;
- comparer les caractéristiques extraites avec un critère, le critère étant représentatif d'un remplissage insuffisant du réservoir ;
- en fonction de la comparaison, déterminer la survenue d'une quantité insuffisante de liquide dans le réservoir.

Le critère représentatif de la quantité insuffisante de liquide est préalablement déterminé au cours d'une phase d'apprentissage. Il peut s'agir :
- d'un seuil de puissance spectrale, dans une bande de fréquence prédéterminée ;
- d'un seuil de variation de la puissance spectrale dans une bande de fréquence prédéterminée, et durant une période temporelle déterminée ;
- d'un seuil d'intensité du son ou d'un seuil de variation de l'intensité du son.

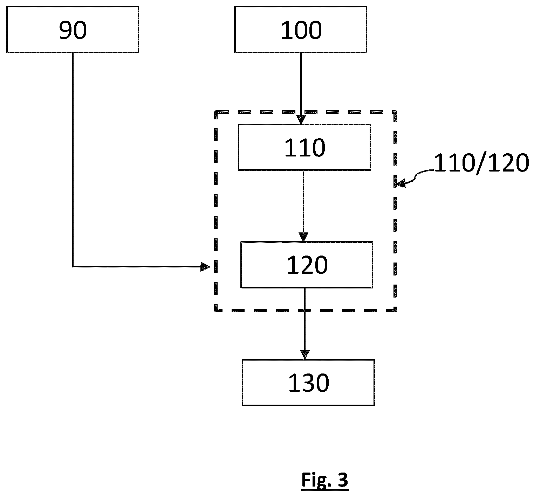

Selon une possibilité, le traitement du son résultant du microphone est effectué par un algorithme d'intelligence artificielle à apprentissage supervisé, comme décrit par la suite en lien avec la figure 3. Le critère est donc « implicite », au sens où il est pris en compte dans le paramétrage de l'algorithme. La sortie de l'algorithme peut être une détection ou une non détection d'un défaut de remplissage du réservoir.

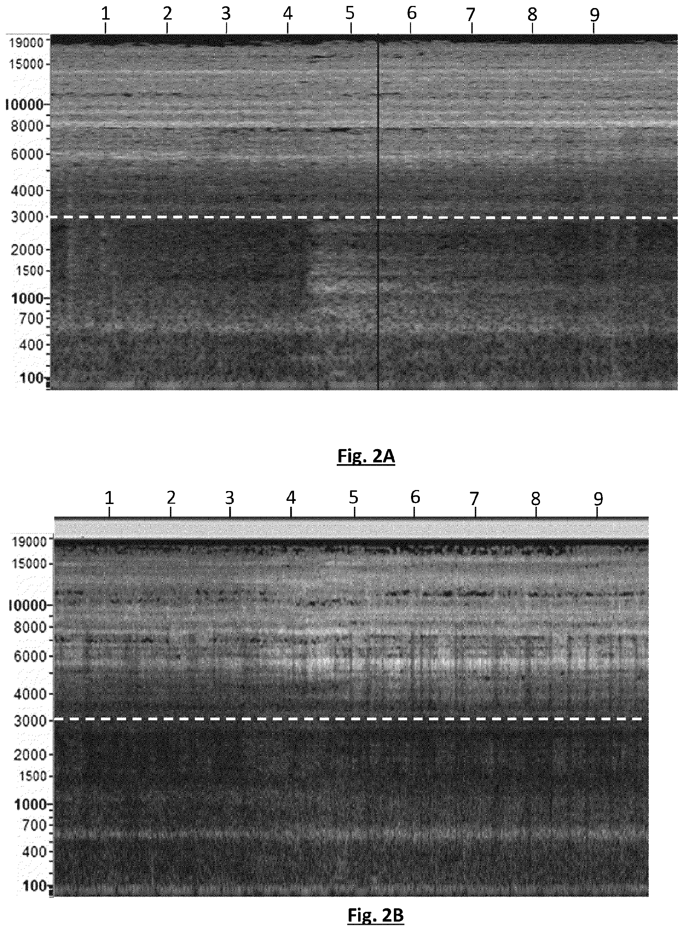

La figure 2A représente un spectrogramme de sons enregistrés au cours d'un essai durant duquel un utilisateur a utilisé un nébuliseur relié à un masque facial. Au cours de l'essai, une quantité suffisante de liquide était présente dans le réservoir.

L'axe des abscisses correspond au temps (unité : seconde). L'axe des ordonnées correspond à la fréquence (Hz). Le niveau de gris correspond à la puissance spectrale.

Sur la figure 2A, on observe que dans une bande de fréquence comprise entre 3000 Hz et 20000 Hz, la puissance spectrale est relativement stable. Cela signifie que l'intensité du signal acoustique par le nébuliseur est relativement stable dans le temps dans cette bande de fréquence.

La figure 2B représente un spectrogramme de sons enregistrés au cours d'un essai similaire au précédent, lorsque la quantité de liquide dans le réservoir était insuffisante. Les axes des abscisses et des ordonnées, ainsi que les niveaux de gris, sont similaires à ceux de la figure 2A. On observe que dans la bande de fréquence de 3000 Hz- 20000 Hz précédemment citée, la puissance spectrale subit des variations, en particulier entre 3000 Hz et 8000 Hz. Dans cette plage de fréquence, on observe des variations de la puissance spectrale en fonction du temps. Ces variations apparaissent, sur la figure 2B, sous la forme de traits verticaux sombres. Ainsi, sur une période temporelle Δt prédéterminée, une variation de la puissance spectrale, supérieure à un seuil de variation prédéfini, est détectée. La période temporelle Δt est par exemple de 1 seconde. Chaque puissance spectrale peut être mesurée selon une fréquence d'échantillonnage de 0.1 s.

Le seuil de variation, la bande de fréquence et la période temporelle peuvent être ajustés au cours d'une période d'apprentissage. On comprend que le seuil de variation et la bande spectrale sont à ajuster en fonction du type de nébuliseur ou du type de microphone utilisé.

Ainsi, selon cet exemple, on extrait à chaque instant une caractéristique du son produit par le nébuliseur, la caractéristique étant ici une variation d'une puissance spectrale au cours d'une période temporelle comportant ledit instant. La caractéristique extraite est comparée à un critère, ce dernier étant le seuil de variation précédemment évoqué. En fonction de la comparaison, une quantité insuffisante de liquide dans le réservoir est détectée ou non.

Selon une variante, un critère formant un seuil de puissance spectrale peut être établi, pour une ou plusieurs fréquences supérieures à 3000 Hz. Lorsqu'à au moins une desdites fréquences, la puissance spectrale mesurée est inférieure au seuil, un défaut de remplissage peut être détecté. Le procédé est plus robuste en considérant simultanément différentes bandes spectrales.

Sur les figures 2A et 2B, le seuil de 3000 Hz a été représenté par une ligne en pointillés.

La figure 3 montre les principales étapes du traitement des sons mis en œuvre par l'unité de traitement.
- au cours d'une étape 100, l'unité de traitement 3 reçoit le son détecté par le microphone ;
- au cours d'une étape 110, l'unité de traitement 3 effectue une extraction d'une ou plusieurs caractéristiques du son détecté;
- au cours d'une étape 120, l'unité de traitement 3 prend en compte un critère, préalablement déterminé au cours d'une phase d'apprentissage 90. La phase d'apprentissage consiste à effectuer des essais pour un type de nébuliseur, avec un des niveaux de remplissage suffisants et insuffisants, de façon à déterminer le ou les critères correspondant à un niveau de remplissage insuffisant.
- au cours d'une étape 130, l'unité de traitement 3 détecte un niveau de remplissage insuffisant lorsque au moins une caractéristique extraite, ou lorsque chaque caractéristique extraite, correspond à un critère défini au cours de l'étape 120. En cas de détection d'une quantité insuffisante de liquide, un signal d'alarme peut être généré, de façon à alerter l'utilisateur ou une équipe de surveillance de l'utilisateur.

Selon une possibilité, la quantité insuffisante de remplissage est détectée en mettant en œuvre un algorithme d'intelligence artificielle à apprentissage supervisé, par exemple un réseau de neurones. L'algorithme est alimenté par soit par le son enregistré par le microphone, soit par des caractéristiques du son préalablement extraites.

La sortie de l'algorithme est une quantité, suffisante ou insuffisante, du remplissage. Le paramétrage de l'algorithme est effectué durant la phase d'apprentissage, en prenant en compte des sons enregistrés respectivement avec une quantité de liquide suffisante ou insuffisante. Selon un tel mode de réalisation, le critère est implicitement pris en compte dans l'algorithme. Les étapes 110 et 120 sont fusionnées en une même étape 110/120, qui correspond à la mise en œuvre de l'algorithme.

L'invention pourra être mise en œuvre pour la surveillance d'utilisateurs d'un nébuliseur, aussi bien dans un environnement médical qu'au domicile. Elle ne nécessite pas d'équipements encombrants ou coûteux, ce qui la rend particulièrement adaptée à une utilisation à domicile.

Un autre avantage est que l'invention ne nécessite pas une instrumentation spécifique du nébuliseur, comme par exemple un détecteur de niveau de liquide dans le réservoir. L'invention s'applique à différents types de nébuliseurs, différents du nébuliseur pneumatique décrit en lien avec la figure 1. De préférence, l'invention implique une phase d'apprentissage pour chaque type de nébuliseur, de façon à affiner les caractéristiques sonores à extraire ainsi que le ou les critères à prendre en compte.

## Revendications

1. Procédé de détection d'une quantité insuffisante de liquide dans un réservoir (11) d'un nébuliseur (10), le nébuliseur étant configuré pour former un aérosol à partir du liquide présent dans le réservoir, de façon à administrer un principe actif, présent dans le liquide, à un utilisateur, le procédé comportant :
- a) détection, par un microphone (2), d'un son produit, en différents instants, par la formation de l'aérosol ;
- b) traitement du son détecté par une unité de traitement (3), à chaque instant de façon à en extraire au moins une caractéristique du son;
- c) en fonction de chaque caractéristique extraite lors de l'étape b), détection, par l'unité de traitement, d'une quantité insuffisante de liquide dans le réservoir pour l'administration du principe actif ;
le procédé étant **caractérisé en ce que** lors de l'étape b), la caractéristique du son est extraite dans une bande de fréquence comprise entre 3000 Hz et 20000 Hz.

2. Procédé selon la revendication 1, dans lequel lors de l'étape b), la caractéristique du son est extraite dans une bande de fréquence comprise entre 3000 Hz et 8000 Hz.

3. Procédé selon la revendication 1 ou la revendication 2, dans lequel l'étape c) comporte :
- c1) prise en compte d'au moins un critère préalablement mémorisé ;
- c2) confrontation de chaque caractéristique extraite lors de l'étape b) au critère pris en compte lors de la sous-étape c1).

4. Procédé selon l'une quelconque des revendications précédentes, dans lequel le traitement comporte :
- sélection d'au moins une bande de fréquence du son ;
- détermination d'une caractéristique dans la bande de fréquence sélectionnée.

5. Procédé selon la revendication 4, dans lequel :
- la caractéristique est une puissance spectrale ;
- le critère est un seuil de puissance spectrale, de façon que la quantité insuffisante de liquide est détectée lorsque la puissance spectrale, dans la bande de fréquence sélectionnée, franchit le seuil de puissance spectrale.

6. Procédé selon la revendication 5, dans lequel :
- la caractéristique est une variation de puissance spectrale ;
- le critère est un seuil de variation puissance spectrale, de façon que la quantité insuffisante de liquide est détectée lorsque la variation de puissance spectrale, dans la bande de fréquence sélectionnée, et dans un intervalle temporel prédéterminé, franchit le seuil de variation de la puissance spectrale.

7. Procédé selon l'une quelconque des revendications précédentes, dans lequel :
- l'unité de traitement met en œuvre un algorithme d'intelligence artificielle à apprentissage supervisé ;
- l'algorithme d'intelligence artificielle à apprentissage supervisé est paramétré par une phase d'apprentissage prenant en compte des sons détectés en l'absence et en présence d'une anomalie.

8. Dispositif de détection d'une quantité insuffisante de liquide dans un réservoir d'un nébuliseur, le nébuliseur étant configuré pour former un aérosol à partir du liquide présent dans le réservoir, le dispositif comportant :
- un microphone (10), configuré pour enregistrer des sons produits par la formation de l'aérosol;
- une unité de traitement (11), programmée pour recevoir les sons enregistrés par le microphone, et pour mettre en œuvre les étapes b) et c) d'un procédé selon l'une quelconque des revendications précédentes.

## Patentansprüche

1. Verfahren zum Erkennen einer unzureichenden Flüssigkeitsmenge in einem Behälter (11) eines Verneblers (10), wobei der Vernebler dazu ausgelegt ist, aus der im Behälter vorhandenen Flüssigkeit ein Aerosol zu bilden, um einem Anwender einen in der Flüssigkeit enthaltenen Wirkstoff zu verabreichen, wobei das Verfahren Folgendes aufweist:
- a) Erkennung eines Geräusches, das zu verschiedenen Zeitpunkten durch die Bildung des Aerosols entsteht, mittels eines Mikrofons (2);
- b) Verarbeitung des von einer Verarbeitungseinheit (3) erfassten Geräusches zu jedem Zeitpunkt, um daraus mindestens eine Geräuschcharakteristik zu extrahieren;
- c) abhängig von jeder in Schritt b) extrahierten Charakteristik, Erkennung einer für die Verabreichung des Wirkstoffs unzureichenden Flüssigkeitsmenge im Behälter durch die Verarbeitungseinheit;
wobei das Verfahren **dadurch gekennzeichnet ist, dass** in Schritt b) die Geräuschcharakteristik in einem Frequenzband zwischen 3000 Hz und 20000 Hz extrahiert wird.

2. Verfahren nach Anspruch 1, wobei in Schritt b) die Geräuschcharakteristik in einem Frequenzband zwischen 3000 Hz und 8000 Hz extrahiert wird.

3. Verfahren nach Anspruch 1 oder Anspruch 2, wobei der Schritt c) Folgendes aufweist:
- c1) Berücksichtigung mindestens eines zuvor gespeicherten Kriteriums;
- c2) Abgleich jeder in Schritt b) extrahierten Charakteristik mit dem in Unterschritt c1) berücksichtigten Kriterium.

4. Verfahren nach einem der vorhergehenden Ansprüche, wobei die Verarbeitung Folgendes aufweist:
- Auswahl mindestens eines Geräuschfrequenzbandes;
- Bestimmung einer Charakteristik im ausgewählten Frequenzband.

5. Verfahren nach Anspruch 4, wobei:
- die Charakteristik eine spektrale Leistung ist;
- das Kriterium ein Schwellenwert für die spektrale Leistung ist, sodass eine unzureichende Flüssigkeitsmenge erkannt wird, sobald die spektrale Leistung im ausgewählten Frequenzband den Schwellenwert überschreitet.

6. Verfahren nach Anspruch 5, wobei:
- die Charakteristik eine Schwankung der spektralen Leistung ist;
- das Kriterium ein Schwellenwert für die Schwankung der spektralen Leistung ist, sodass eine unzureichende Flüssigkeitsmenge erkannt wird, sobald die Schwankung der spektralen Leistung im ausgewählten Frequenzband und innerhalb eines vorgegebenen Zeitintervalls den Schwellenwert für die Schwankung der spektralen Leistung überschreitet.

7. Verfahren nach einem der vorhergehenden Ansprüche, wobei:
- die Verarbeitungseinheit einen Algorithmus der künstlichen Intelligenz mit überwachtem Lernen anwendet;
- der Algorithmus der künstlichen Intelligenz mit überwachtem Lernen durch eine Lernphase parametrisiert wird, bei der Geräusche berücksichtigt werden, die sowohl in Abwesenheit als auch in Anwesenheit einer Anomalie erfasst wurden.

8. Vorrichtung zum Erkennen einer unzureichenden Flüssigkeitsmenge in einem Behälter eines Verneblers, wobei der Vernebler dazu ausgelegt ist, aus der im Behälter vorhandenen Flüssigkeit ein Aerosol zu bilden, wobei die Vorrichtung Folgendes aufweist:
- ein Mikrofon (10), das dazu ausgelegt ist, durch die Aerosolbildung erzeugte Geräusche aufzuzeichnen;
- eine Verarbeitungseinheit (11), die dazu programmiert ist, die vom Mikrofon aufgezeichneten Geräusche zu empfangen und die Schritte b) und c) eines Verfahrens gemäß einem der vorhergehenden Ansprüche auszuführen.

## Claims

1. A method for detecting an insufficient quantity of liquid in a reservoir of a nebulizer (10), the nebulizer being configured to form an aerosol from the liquid present in the reservoir, so as to deliver an active ingredient, contained in the liquid, to a user, the method comprising :
- a) detecting, by a microphone (2), of a sound produced, at different times, by the formation of the aerosol;
- b) processing the detected sound by a processing unit (3), at each time, so as to extract at least one sound feature;
- c) based on each sound feature extracted in step b), detecting, by the processing unit, an insufficient quantity of liquid in the reservoir for delivery of the active aerosol;
the method being **characterized in that** in step b), the sound feature is extracted in a frequency band between 3000 Hz and 20000 Hz.

2. The method according to claim 1, wherein in step b), the sound feature is extracted in a frequency band between 3000 Hz and 8000 Hz.

3. The method according to claim 1 or claim 2, wherein step c) comprises :
- c1) taken into account at least one previously stored criterion ;
- c2) comparing each feature extracted in step b) with the criterion taken into account in sub-step c1).

4. The method according to any one of the preceding claims, wherein the processing comprises :
- selecting at least one sound frequency band ;
- determining a feature in the selected frequency band.

5. The method according to claim 4, wherein :
- the feature is a spectral power ;
- the criterion is a spectral power threshold, so that the insufficient quantity of liquid is detected when the spectral power in the selected frequency band crosses the spectral power threshold.

6. The method according to claim 5, wherein :
- the feature is a variation in spectral power;
- the criterion is a spectral power variation threshold, so that the insufficient quantity of liquid is detected when the variation in spectral power, in the selected frequency band and within a predetermined time interval, crosses the spectral power variation threshold.

7. The method according to any one of the preceding claims, wherein :
- the processing unit implements a supervised learning artificial intelligence algorithm;
- the supervised learning artificial intelligence algorithm is parameterized by a training phase taking into account sounds detected in the absence and presence of an anomaly.

8. A device for detecting an insufficient quantity of liquid in a reservoir of a nebulizer, the nebulizer being configured to form an aerosol from the liquid present in the reservoir, the device comprising:
- a microphone (10), configured to record sounds produced by aerosol formation;
- a processing unit (11), programmed to receive the sounds recorded by the microphone, and to implement steps b) and c) of the method according to any of the preceding claims.
